# EUROPEAN PATENT SPECIFICATION

(11) **EP 1 073 651 B1**
(45) Date of publication and mention of the grant of the patent: **25.09.2002**
(21) Application number: 99922739.0
(22) Date of filing: 28.04.1999
(51) Int. Cl.: C07D 401/06, A61K 31/445, C07D 401/04

(54) **INDOLYL DERIVATIVES AS SEROTONERGIC AGENTS**
INDOLYLDERIVATE ALS SEROTONERGISCHE WIRKSTOFFE
DERIVES D'INDOLYLE EN TANT QU'AGENTS SEROTONINERGIQUES

(30) Priority: 29.04.1998 US 69043
(43) Date of publication of application: 07.02.2001
(73) Proprietor: Wyeth, Madison, New Jersey 07940-0874 (US)
(72) Inventor: KELLY, Michael, Gerard, Plainsboro, NJ 08536 (US); KANG, Young, Hee, Robbinsville, NJ 08691 (US)
(74) Representative: Connelly, Michael John
(86) International application number: US9909181
(87) International publication number: WO99055695

(56) References cited:
- EP-A- 0 496 222
- WO-A-99/20621
- FR-A- 2 675 801
- US-A- 4 954 502

## Description

This invention relates to novel compounds useful as serotonergic agents. More particularly, this invention concerns indolyl compounds which are useful as serotonergic agents, particularly as serotonin re-uptake inhibitors.

### Background to the Invention

Depression is a psychiatric condition thought to be associated with decreased serotonin release. Most antidepressant agents potentiate the effects of serotonin by blocking the termination of its activity through re-uptake into nerve terminals.

U.S. Pat. No. 5,342,845 (Chokai et al.) teaches indole carboxamide derivatives of the general formula: 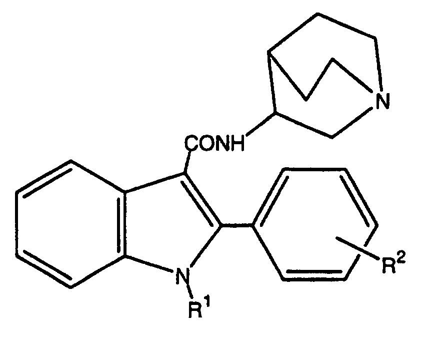 wherein R¹ is lower alkyl and R² is selected from H, halogen, lower alkyl or lower alkoxy, useful for gastro-intestinal motor activity regulation, antimigraine, antipsychotic or antianxiety drugs.

U.S. Pat. No. 5,614,523 (Audia et al.) teaches hetero-oxy alkanamines which are effective in treatments for conditions related to or affected by the reuptake of serotonin and by the serotonin 1_{A} receptor.

U.S. Pat. No. 5,693,655 (Bottcher et al.) discloses 3-indolylpiperidines which exhibit action on the central nervous system, particularly dopamine-agonistic or dopamine-antagonistic actions.

U.S. Pat. No. 5,670,511 (Marz et al.) claims indolepiperidine derivatives also having dopamine agonistic or antagonistic action, the compounds having the general formula below, wherein R² is selected from -NH-CO-Ar, -NH-SO₂-Ar, or D, wherein D is as also shown below:

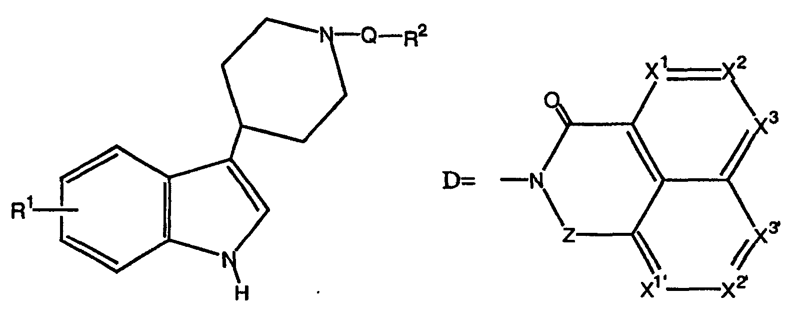

U.S. Pats. Nos. 5,541,794 and 5,654,324 (both to Booher et al.) claim 6-heterocyclic-4-amino-1,2,2a,3,4,5-hexahydrobenz-[cd] indoles useful in modifying the function of serotonin in mammals.

U.S. Pat. No. 5,654,320 (Catlow et al.) also disclose indazolecarboxamides useful as antagonists and partial agonists for the serotonin 5-HT₄ receptor and treatments for dysfunctions thereof.

WO 99/20621 was published on 29 April 1999 with a claim to priority from US Patent Application 60/063,493 filed 22 October 1997. The publication discloses a class of compounds that inhibit the reuptake of serotonin and include compounds of the formula 17 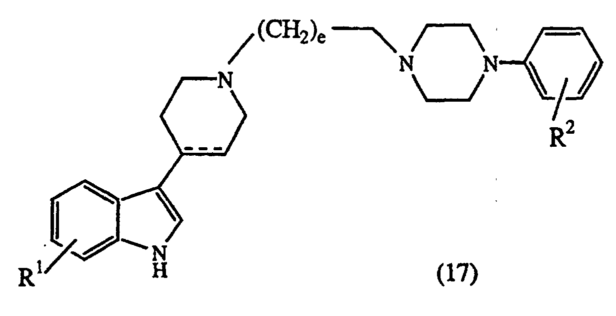 where e is an integer from 3 to 7 both inclusive;
R¹ and R² are each independently hydrogen, halo, -C₁-C₆ alkyl or -C₁-C₆ alkoxy; and
----represents a double or single bond;
or a pharmaceutically acceptable salt or solvate thereof.

This invention relates to novel indolyl derivatives, to processes for their preparation, to pharmaceutical compositions containing them and to their use in therapy. The novel compounds are useful for the treatment of central nervous system disorders, particularly depression, by virtue of their ability to inhibit the uptake of serotonin.

### Summary of the Present Invention

Compounds of the present invention are represented by the general formula (1) 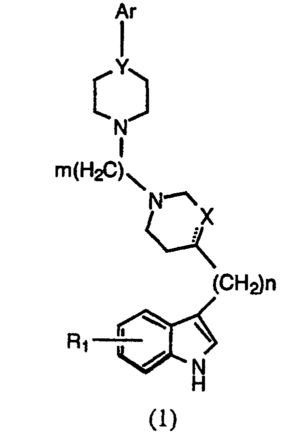 wherein:
R₁ is selected from H, OH, OR₂, F, Cl, Br, or I;
R₂ is lower alkyl (C₁ to C₆);
n is selected from 0, 1 or 2;
X is CH or CH₂;
m is an integer selected from 2, 3 or 4;
Y is N or CH;
Ar is aryl or heteroaryl, both optionally substituted by from one to three groups selected from F, Cl, Br, I, -OH, -CN, lower alkoxy (C₁ to C₆) or lower alkyl (C₁ to C₆);
or a pharmaceutically acceptable salt thereof.

The compounds of the invention exclude the compounds of formula 17 of WO 99/20621 and their pharmaceutically acceptable addition salts. The compounds are therefore subject to the proviso that where Ar is 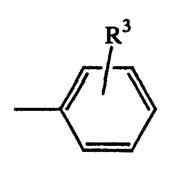 in which R³ is hydrogen, halo, C₁-C₆ alkyl or C₁-C₆ alkoxy and R¹ is hydrogen, halo or C₁-C₆ alkoxy whilst n is 0 and m is 4, then Y is other than N.

A preferred subset of compounds of this invention are those in which Y is nitrogen and R₁, R₂, n, X, m, Y and Ar are as defined above.

The preferred aryl or heteroaryl groups comprising Ar in the groups above are phenyl, benzodioxane, indole bonded to the Y moiety in the indole 4- or 7-position, pyridine, 2-pyrimidine, thiophene, furan or pyrrole. The most preferred of these groups are phenyl, benzodioxole-5-yl, and 2-pyrimidine.

The pharmaceutically acceptable salts are the acid addition salts which can be formed from a compound of the above general formula and a pharmaceutically acceptable inorganic acid, such as phosphoric, sulfuric, hydrochloric, hydrobromic, citric, maleic, furmaric, acetic, lactic or methanesulfonic acid.

### Detailed Description of the Invention

Compounds of the present invention may be prepared using conventional methods. For example, the appropriately substituted indole (A) can be coupled with a chloroalkyl-substituted arylpiperazine or arylpiperidine (B) using a base such as diisopropylethylamine. The product can then be used to form a pharmaceutically acceptable salt.

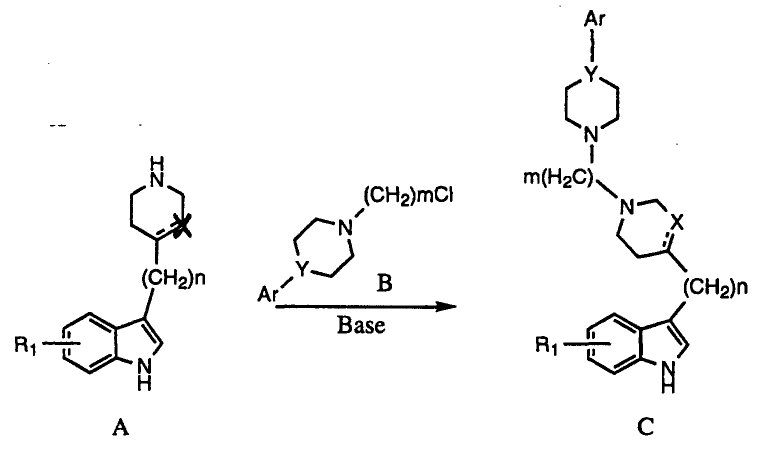

The preparation of the appropiately substituted 3-(4-piperidinyl)indoles and 3-(4-tetrahydro pyridinyl) indoles can be acheived by known and convential methods. For example, the reaction of an optionally substituted indole (D) with 4-piperidone (E) affords the 3-(4-tetrahydropyridinyl)indole (F). This can be reduced using standard catalytic hydrogenation methodology to afford a 3-(4-piperidinyl)indole (G).

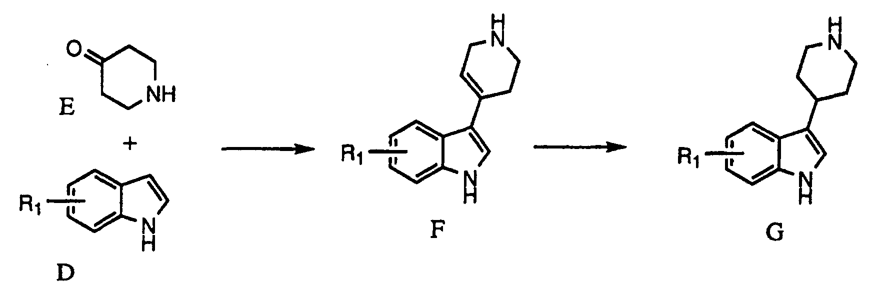

The preparation of the appropiately substituted 3-(4-piperidinylmethyl)indoles (H) and 3-(4-tetrahydropyridinylmethyl)indoles (I) can also be achieved by known and convential methods. Such methodology is described in C. Gueremy et al., J. Med. Chem., 1980, 23, 1306-1310, J-L. Malleron et al., J. Med. Chem., 1993, 36, 1194-1202 and J. Bergman, J. Heterocyclic. Chem., 1970, 1071-1076.

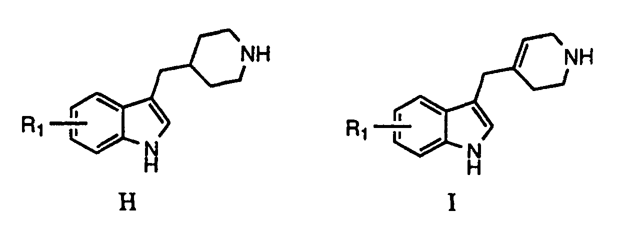

Compounds of the present invention inhibit with very high affinity the binding of paroxetine to the serotonin transporter and, consequently, are useful as antidepressant and anxiolytic agents for the treatment of central nervous system disorders such as depression, anxiety, sleep disorders, sexual dysfunction, alcohol and cocaine addiction, cognition enhancement and related problems. In addition, compounds of the present invention may be used in conjunction with an agonist or antagonist of the serotonin-1 receptor (5-HT1) to aid or enhance the compounds biological properties. Such compositions may be useful for the above mentioned disorders in addition to the treatment of Alzheimer's disease, Parkinson's disease, schizophrenia, obesity and migraine.

It is understood that the therapeutically effective dosage to be used in the treatment of a specific psychosis must be subjectively determined by the attending physician. Variables involved include the specific psychosis or state of anxiety and the size, age and response pattern of the patient. The novel method of the invention for treating conditions related to or are affected by the reuptake of serotonin comprise administering to warm-blooded animals, including humans, an effective amount of at least one compound of this invention or a non-toxic, pharmaceutically acceptable addition salt thereof. The compounds may be administered orally, rectally, parenterally, or topically to the skin and mucosa. The usual daily dose is depending on the specific compound, method of treatment and condition treated. An effective dose of 0.01 - 1000mg/Kg may be used for oral application, preferably 0.5 - 500 mg/Kg, and an effective amount of 0.1 - 100 mg/Kg may be used for parenteral application, preferably 0.5 - 50 mg/Kg.

The present invention also includes pharmaceutical compositions containing a compound of this invention, or a pharmaceutically acceptable salt thereof, and one or more pharmaceutically acceptable carriers or excipients.

Applicable solid carriers or excipients can include one or more substances which may also act as flavoring agents, lubricants, solubilizers, suspending agents, fillers, glidants, compression aids, binders or tablet-disintergrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be either liquid or solid composition form.

Preferably the pharmaceutical composition is in unit dosage form, e.g. as tablets or capsules. In such form, the composition is sub-divided in unit dose containing appropriate quantities of the active ingredient; the unit dosage forms can be packaged compositions, for example packeted powders, vials, ampoules, prefilled syringes or sachets containing liquids. The unit dosage form can be, for example, a capsule or tablet itself, or it can be the appropriate number of any such compositions in package form.

The affinity of drugs for the serotonin transporter was determined by assessing the ability of agents to displace specifically bound 3H-paroxetine binding from rat cortical membranes. A protocol similar to that used by Cheetham et al. (Neuropharmacol. 32:737, 1993) was used to determine the affinity of compounds for the serotonin transporter. Briefly, frontal cortical membranes prepared from male S.D. rats were incubated with ³H-paroxetine (0.1 nM) for 60 min at 25°C. All tubes also contained either vehicle, test compound (one to eight concentrations), or a saturating concentration of fluoxetine (10 µM) to define specific binding. All reactions are terminated by the addition of ice cold Tris buffer followed by rapid filtration using a Tom Tech filtration device to separate bound from free ³H-paroxetine. Bound radioactivity was quantitated using a Wallac 1205 Beta Plate® counter. Nonlinear regression analysis was used to determine IC₅₀ values which were converted to Ki values using the method of Cheng and Prusoff (Biochem. Pharmacol. 22: 3099, 1973); Ki = IC50/((Radioligand conc.)/(1 + KD)). Nonspecific binding was determined using fluoxetine. Using this assay, the following Ki's were determined for a series of standard serotonin uptake inhibitors.

| **Compound** | **Inhibition of [3H]-Paroxetine binding** |
|---|---|
| | **Ki (nM)** |
| Clomipramine | 0.18 |
| Fluoxetine | 4.42 |
| Imipramine | 17.6 |
| Zimelidine | 76.7 |

The results for a number of examples of compounds of formula 1 in this standard experimental test procedure were as follows:

| **Compound** | **Inhibition of [3H]-Paroxetine binding** |
|---|---|
| | **Ki (nM)** |
| Example 1 | 4.8 |
| Example 3 | 1.2 |
| Example 6 | 10.0 |
| Example 7 | 19.0 |

The following non-limiting specific examples are included to illustrate the synthetic procedures which may be used for preparing compounds of the formula 1. In these examples, all chemicals and intermediates are either commercially available or can be prepared by standard procedures found in the literature or are known to those skilled in the art of organic synthesis.

### Example 1 5-Fluoro-3-(1-{2-[4-(2-methoxyphenyl)piperazin-1-yl] ethyl}piperidin-4-ylmethyl)-1H-indole

Pulverized potassium carbonate (0.76 g, 5.5 mmole) and potassium iodide (0.97 g, 5.5 mmole) was added to a mixture of 4-(5-fluoro-1H-indol-3-ylmethyl)piperidine (1.16 g, 5.0 mmole) and 1-(2-chloroethyl)-4-(2-methoxyphenyl)piperazine (1.27 g, 5.0 mmole) in 25 ml of acetonitrile. The resulting mixture was heated to reflux under nitrogen for 5 hours. After cooling, it was diluted with water (150 ml) and the product extracted into ethyl acetate (50 ml). The organic layer was washed with water (50 ml), brine (50 ml), and after drying over sodium sulfate, filtration and concentration *in vacuo* afforded the required product as a white solid (1.8 g, 80 %). Treatment with an excess of 1M etheral hydrochloric acid gave the acid addition salt, which was recrystallized from methanol.
m.p. 253-254 °C
Elemental Analysis for: C27H35FN4O. 2HCl
Calculated: C, 61.95; H, 7.13; N, 10.68
Found: C, 61.84; H, 7.18; N, 10.61

### Example 2 5-Fluoro-3-(1-{2-[4-(2-methoxyphenyl)piperazin-1-yl] ethyl}piperidin-4-yl)-1H-indole

Pulverized potassium carbonate (0.35 g, 2.5 mmole) and potassium iodide (0.44 g, 2.5 mmole) was added to a mixture of 4-(5-fluoro-1H-indol-3-yl)piperidine (0.5 g, 2.3 mmole) and 1-(2-chloroethyl)-4-(2-methoxyphenyl)piperazine (0.58 g, 2.3 mmole) in 20 ml of acetonitrile. The resulting mixture was heated to reflux under nitrogen for 3 hours. After cooling, it was diluted with water (150 ml) and the product extracted into ethyl acetate (50 ml). The organic layer was washed with water (50 ml), brine (50 ml), and after drying over sodium sulfate, filtration and concentration *in vacuo* afforded the required product as a white solid (0.83 g, 83 %). Treatment with an excess of 1M etheral hydrochloric acid gave the acid addition salt, which was recrystallized from methanol.
m.p. 251-252 °C
Elemental Analysis for: C26H33FN4O. 2HCl. 0.25H2O
Calculated: C, 60.76; H, 6.96; N, 10.90
Found: C, 60.79; H, 7.09; N, 10.85

### Example 3 5-Fluoro-3-(1-{3-[4-(2-methoxyphenyl)piperazin-1-yl] propyl}piperidin-4-ylmethyl)-1H-indole

Pulverized potassium carbonate (0.33 g, 2.4 mmole) and potassium iodide (0.40 g, 2.4 mmole) was added to a mixture of 4-(5-fluoro-1H-indol-3-yl)methylpiperidine (0.5 g, 2.3 mmole) and 1-(2-chloroethyl)-4-(2-methoxyphenyl)piperazine (0.58 g, 2.3 mmole) in 20 ml of acetonitrile. The resulting mixture was heated to reflux under nitrogen for 3 hours. After cooling, it was diluted with water (150 ml) and the product extracted into ethyl acetate (50 ml). The organic layer was washed with water (50 ml), brine (50 ml), and after drying over sodium sulfate, filtration and concentration *in vacuo* afforded the required product as a white solid (0.78 g, 76 %). Treatment with an excess of 0.25M ethanolic fumaric acid solution gave the acid addition salt, which was recrystallized from ethanol/diethyl ether to afford the title product as white needles.
m.p. 156-157 °C
Elemental Analysis for: C28H37FN4O. 2C4H4O4
Calculated: C, 62.06; H, 6.51; N, 8.04
Found: C, 61.76; H, 6.68; N, 7.87

### Example 4 5-Fluoro-3-(1-{3-[4-(2-methoxyphenyl)piperazin-1-yl] propyl}piperidin-4-yl)-1H-indole

The title compound was prepared using the procedure outlined in examples 1-3 above. The product was purified by silica gel column chromatography, and was isolated in 68% yield. Its fumaric acid salt was obtained as a fine white powder.
m.p. 200 °C
Elemental Analysis for: C27H35FN4O. 2C4H4O4
Calculated: C, 61.57; H, 6.35; N, 8.21
Found: C, 61.59; H, 6.53; N, 8.12

### Example 5 3-(1-{2-[4-(2-methoxyphenyl)piperazin-1-yl]ethyl} piperidin-4-yl)-1H-indole

The title compound was prepared using the procedure outlined in examples 1-3 above. The product was purified by silica gel column chromatography, and was isolated in 96% yield as a white solid. Its fumaric acid salt was prepared as reported in example 3 and was obtained as a fine white powder.
m.p. 212-213 °C
Elemental Analysis for: C26H34N4O. 2C4H4O4
Calculated: C, 62.76; H, 6.51; N, 8.61
Found: C, 62.82; H, 6.48; N, 8.63

### Example 6 3-(1-{2-[4-(2-methoxyphenyl)piperazin-1-yl]ethyl} 1,2,3,6-tetrahydropyridin-4-yl)-1H-indole

The title compound was prepared using the procedure outlined in examples 1-3 above. The product was purified by silica gel column chromatography, and was isolated in 64% yield as a light yellow solid. Its fumaric acid salt was prepared as reported in example 3.
m.p. 189 °C
Elemental Analysis for: C26H32N4O. 0.5C4H4O4
Calculated: C, 70.82; H, 7.26; N, 11.73
Found: C, 70.50; H, 7.27; N, 11.68

### Example 7 5-Fluoro-3-(1-{2-[4-(2-methoxyphenyl)piperazin-1-yl]ethyl} 1,2,3,6-tetrahydropyridin-4-yl)-1H-indole

The title compound was prepared using the procedure outlined in examples 1-3 above. The product was purified by silica gel column chromatography, and was isolated in 69% yield as a yellow solid. Its fumaric acid salt was prepared as reported in example 3 and was obtained as a fine white solid.
m.p. 198-199 °C
Elemental Analysis for: C26H31FN4O. 2C4H4O4
Calculated: C, 61.25; H, 5.90; N, 8.40
Found: C, 61.37; H, 5.87; N, 8.46

### Example 8 5-Fluoro-3-(1-{2-[4-(2-fluorophenyl)piperazin-1-yl]ethyl} 1,2,3,6-tetrahydropyridin-4-yl)-1H-indole

The title compound is prepared using the procedure outlined in examples 1-3 above. The product may be purified by silica gel column. Its fumaric acid salt can be prepared as reported in example 3.

### Example 9 5-Fluoro-3-(1-{2-[4-(indol-4-yl)piperazin-1-yl]ethyl} 1,2,3,6-tetrahydropyridin-4-yl)-1H-indole

The title compound is prepared using the procedure outlined in examples 1-3 above. The product may be purified by silica gel column chromatography its fumaric acid salt prepared as reported in example 3.

## Claims

1. A compound according to Formula 1: 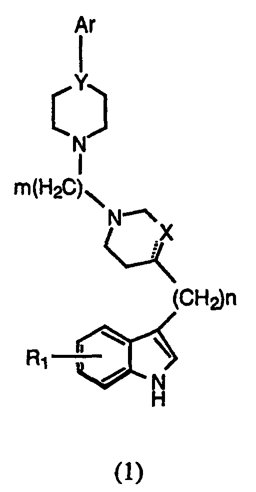 wherein;
R₁ is selected from H, OH, OR₂ or halogen (F, Cl, Br, I);
R₂ is lower alkyl (C₁ to C₆);
n is selected from 0, 1 or 2;
X is CH or CH₂;
m is selected from 2, 3 or 4;
Y is N or CH;
Ar is aryl or heteroaryl, both optionally substituted by from one to three groups selected from F, Cl, Br, I, -OH, -CN, lower alkoxy (C₁ to C₆) or lower alkyl (C₁ to C₆);
or a pharmaceutically acceptable salt thereof; subject to the proviso that where Ar is 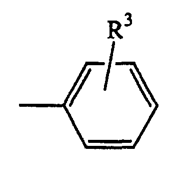 in which R³ is hydrogen, halo, C₁-C₆ alkyl or C₁-C₆ alkoxy and R¹ is hydrogen, halo or C₁-C₆ alkoxy whilst n is 0 and m is 4, then Y is other than N.

2. A compound of claim 1, in which Y is nitrogen.

3. A compound of claim 1 or 2 in which Ar is aryl or heteroaryl selected from phenyl, benzodioxane, indole bonded to the Y moiety in the indole 4- or 7-position, pyridine, 2-pyrimidine, thiophene, furan or pyrrole, the aryl or heteroaryl being optionally substituted by from one to three groups selected from F, Cl, Br, I, -OH, -CN, lower alkoxy (C₁ to C₆) or lower alkyl (C₁ to C₆).

4. A compound of claim 1 or 2 in which Ar is aryl or heteroaryl selected from phenyl, benzodioxole-5-yl, and 2-pyrimidine, the aryl or heteroaryl being optionally substituted by from one to three groups selected from F, Cl, Br, I, -OH, -CN, lower alkoxy (C₁ to C₆) or lower alkyl (C₁ to C₆).

5. A compound of claim 1 which is 5-Fluoro-3-(1-{2-[4-(2-methoxyphenyl)piperazin-1-yl]ethyl}piperidin-4-ylmethyl)-1H-indole or a pharmaceutically acceptable salt thereof.

6. A compound of claim 1 which is 5-Fluoro-3-(1-{2-[4-(2-methoxyphenyl)piperazin-1-yl]ethyl}piperidin-4-yl)-1H-indole or a pharmaceutically acceptable salt thereof.

7. A compound of claim 1 which is 5-Fluoro-3-(1-{3-[4-(2-methoxyphenyl)piperazin-1-yl]propyl }piperidin-4-ylmethyl)-1H-indole or a pharmaceutically acceptable salt thereof.

8. A compound of claim 1 which is 5-Fluoro-3-(1-{3-[4-(2-methoxyphenyl)piperazin-1-yl]propyl}piperidin-4-yl)-1H-indole or a pharmaceutically acceptable salt thereof.

9. A compound of claim 1 which is 3-(1-{2-[4-(2-methoxyphenyl)piperazin-1-yl]ethyl} piperidin-4-yl)-1H-indole or a pharmaceutically acceptable salt thereof.

10. A compound of claim 1 which is 3-(1-{2-[4-(2-methoxyphenyl)piperazin-1-yl]ethyl} 1,2,3,6-tetrahydropyridin-4-yl)-1H-indole or a pharmaceutically acceptable salt thereof.

11. A compound of claim 1 which is 5-Fluoro-3-(1-{2-[4-(2-methoxyphenyl)piperazin-1-yl]ethyl}1,2,3,6-tetrahydropyridin-4-yl)-1H-indole or a pharmaceutically acceptable salt thereof.

12. A pharmaceutical composition comprising a pharmaceutically acceptable carrier or excipient and a compound of any one of claims 1 to 11, or a pharmaceutically acceptable salt thereof.

13. A compound as claimed in any one of claims 1 to 11, or a pharmaceutically acceptable salt thereof for use as a pharmaceutical.

14. Use of a compound as claimed in any one of claims 1 to 11 or a pharmaceutically acceptable salt thereof for preparing a medicament for therapeutic use as an inhibitor of the reuptake of serotonin.

15. Use of a compound as claimed in any one of claims 1 to 11 or a pharmaceutically acceptable salt thereof for preparing a medicament for treating depression.

16. Use of a compound as claimed in any one of claims 1 to 11 or a pharmaceutically acceptable salt thereof for preparing a medicament for treating anxiety.

## Patentansprüche

1. Verbindung gemäß Formel 1: 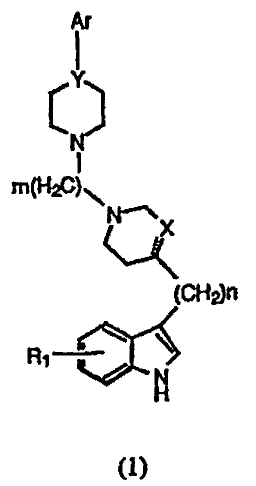 worin;
R₁ ausgewählt wird aus H, OH, OR₂ oder Halogen (F, Cl, Br, I) ;
R₂ nied.-Alkyl (C₁ bis C₆) darstellt;
n ausgewählt wird aus 0, 1 oder 2;
X für CH oder CH₂ steht;
m ausgewählt wird aus 2, 3 oder 4;
Y für N oder CH steht;
Ar für Aryl oder Heteroaryl steht, beides gegebenenfalls substituiert durch eine bis drei Gruppen, ausgewählt aus F, Cl, Br, I, -OH, -CN, nied.-Alkoxy (C₁ bis C₆) oder nied.-Alkyl (C₁ bis C₆);
oder ein pharmazeutisch annehmbares Salz davon; unter der Bedingung, dass wo Ar für 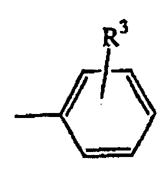 steht, worin R³ Wasserstoff, Halogen, C₁-C₆-Alkyl oder C₁-C₆-Alkoxy darstellt und R¹ Wasserstoff, Halogen oder C₁-C₆-Alkoxy darstellt, während n für 0 steht und m für 4 steht, Y etwas anderes als N darstellt.

2. Verbindung nach.Anspruch 1, worin Y für Stickstoff steht.

3. Verbindung nach Anspruch 1 oder 2, worin Ar für Aryl oder Heteroaryl steht, ausgewählt aus Phenyl, Benzodioxan, Indol, welches in der Indol 4- oder 7-Position an die Y-Komponente gebunden ist, Pyridin, 2-Pyrimidin, Thiophen, Furan oder Pyrrol, wobei das Aryl oder Heteroaryl gegebenenfalls durch eine bis drei Gruppen substituiert sein können, ausgewählt aus F, Cl, Br, I, -OH, -CN, nied.-Alkoxy (C₁ bis C₆) oder nied.-Alkyl (C₁ bis C₆).

4. Verbindung nach Anspruch 1 oder 2, worin Ar für Aryl oder Heteroaryl steht, ausgewählt aus Phenyl, Benzodioxol-5-yl und 2-Pyrimidin, wobei das Aryl oder Heteroaryl gegebenenfalls substituiert sind durch eine bis drei Gruppen, ausgewählt aus F, Cl, Br, I, -OH, -CN, nied.-Alkoxy (C₁ bis C₆) oder nied.-Alkyl (C₁ bis C₆).

5. Verbindung nach Anspruch 1, welche 5-Fluor-3-(1-{2-[4-(2-methoxyphenyl)piperazin-1-yl]ethyl}piperidin-4-ylmethyl)-1Hindol oder ein pharmazeutisch annehmbares Salz davon ist.

6. Verbindung nach Anspruch 1, welche 5-Fluor-3-(1-{2-[4-(2-methoxyphenyl)piperazin-1-yl]ethyl}piperidin-4-yl)-1H-indol oder ein pharmazeutisch annehmbares Salz davon ist.

7. Verbindung nach Anspruch 1, welche 5-Fluor-3-(1-{3-[4-(2-methoxyphenyl)piperazin-1-yl]propyl}piperidin-4-ylmethyl)-1Hindol oder ein pharmazeutisch annehmbares Salz davon ist.

8. Verbindung nach Anspruch 1, welche 5-Fluor-3-(1-{3-[4-(2-methoxyphenyl)piperazin-1-yl]propyl}piperidin-4-yl)-1H-indol oder ein pharmazeutisch annehmbares Salz davon ist.

9. Verbindung nach Anspruch 1, welche 3-(1-{2-[4-(2-Methoxyphenyl)piperazin-1-yl]ethyl}piperidin-4-yl)-1H-indol oder ein pharmazeutisch annehmbares Salz davon ist.

10. Verbindung nach Anspruch 1, welche 3-(1-{2-[4-(2-Methoxyphenyl)piperazin-1-yl]ethyl}1,2,3,6-tetrahydropyridin-4-yl)-1Hindol oder ein pharmazeutisch annehmbares Salz davon ist.

11. Verbindung nach Anspruch 1, welche 5-Fluor-3-(1-{2-[4-(2-methoxyphenyl)piperazin-1-yl]ethyl}1,2,3,6-tetrahydropyridin-4-yl)-1H-indol oder ein pharmazeutisch annehmbares Salz davon ist.

12. Pharmazeutische Zusammensetzung, welche einen pharmazeutisch annehmbaren Träger oder Arzneimittelträger und eine Verbindung nach einem der Ansprüche 1 bis 11 umfasst, oder ein pharmazeutisch annehmbares Salz davon.

13. Verbindung, wie in einem der Ansprüche 1 bis 11 beansprucht, oder ein pharmazeutisch annehmbares Salz davon, zur Verwendung als Pharmazeutikum.

14. Verwendung einer Verbindung, wie in einem der Ansprüche 1 bis 11 beansprucht, oder eines pharmazeutisch annehmbaren Salzes davon, zum Herstellen einer Arznei zur therapeutischen Verwendung als Serotonin-Wiederaufnahmehemmer.

15. Verwendung einer Verbindung, wie in einem der Ansprüche 1 bis 11 beansprucht, oder eines pharmazeutisch annehmbaren Salzes davon, zum Herstellen einer Arznei zum Behandeln von Depression.

16. Verwendung einer Verbindung, wie in einem der Ansprüche 1 bis 11 beansprucht, oder eines pharmazeutisch annehmbaren Salzes davon, zum Herstellen einer Arznei zum Behandeln von Angstzuständen.

## Revendications

1. Composé selon la formule I : 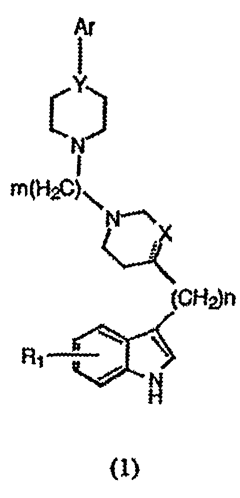 dans laquelle
R₁ est choisi parmi H, OH, OR₂ ou halogène (F, Cl, Br, I) ;
R₂ est alkyle inférieur (en C₁ à C₆) ;
n est choisi parmi 0, 1 ou 2 ;
X est CH ou CH₂ ;
m est choisi parmi 2, 3 ou 4 ;
Y est N ou CH ;
Ar est aryle ou hétéroaryle, tous deux facultativement substitués par un à trois groupements choisis parmi F, Cl, Br, I, -OH, -CN, alkoxy inférieur (en C₁ à C₆) ou alkyle inférieur (en C₁ à C₆) ;
ou un sel pharmaceutiquement acceptable de celui-ci ; soumis à la condition que si Ar est 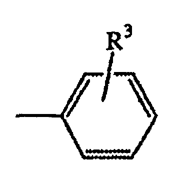 dans laquelle R³ est hydrogène, halogène, alkyle en C₁-C₆ ou alkoxy en C₁-C₆ et R¹ est hydrogène, halogène ou alkoxy en C₁-C₆, pendant que n est 0 et m est 4, alors Y est autre que N.

2. Composé selon la revendication 1 dans lequel Y est azote.

3. Composé selon la revendication 1 ou 2 dans lequel Ar est aryle ou hétéroaryle choisi parmi phényle, benzodioxanne, indole lié à la partie Y dans la position 4 ou 7 de l'indole, pyridine, 2-pyrimidine, thiophène, furanne ou pyrrole, l'aryle ou l'hétéroaryle étant facultativement substitué par un à trois groupements choisis parmi F, Cl, Br, I, -OH, -CN, alkoxy inférieur (en C₁-C₆) ou alkyle inférieur (en C₁-C₆).

4. Composé selon la revendication 1 ou 2 dans lequel Ar est aryle ou hétéroaryle choisi parmi phényle, benzodioxole-5-yle et 2-pyrimidine, l'aryle ou l'hétéroaryle étant facultativement substitué par un à trois groupements choisis parmi F, Cl, Br, I, -OH, -CN, alkoxy inférieur (en C₁-C₆) ou alkyle inférieur (en C₁-C₆).

5. Composé selon la revendication 1 qui est le 5-fluoro-3-(1-{2-[4-(2-méthoxyphényl)pipérazin-1-yl]éthyl}pipéridin-4-ylméthyl)-1H-indole ou un sel pharmaceutiquement acceptable de celui-ci.

6. Composé selon la revendication 1 qui est le 5-fluoro-3-(1-{2-[4-(2-méthoxyphényl)pipérazin-1-yl]éthyl}pipéridin-4-yl)-1H-indole ou un sel pharmaceutiquement acceptable de celui-ci.

7. Composé selon la revendication 1 qui est le 5-fluoro-3-(1-{3-[4-(2-méthoxyphényl)pipérazin-1-yl]propyl}pipéridin-4-ylméthyl)-1H-indole ou un sel pharmaceutiquement acceptable de celui-ci.

8. Composé selon la revendication 1 qui est le 5-fluoro-3-(1-{3-[4-(2-méthoxyphényl)pipérazin-1-yl]propyl}pipéridin-4-yl)-1H-indole ou un sel pharmaceutiquement acceptable de celui-ci.

9. Composé selon la revendication 1 qui est le 3-(1-{2-[4-(2-méthoxyphényl)pipérazin-1-yl]éthyl}-pipéridin-4-yl)-1H-indole ou un sel pharmaceutiquement acceptable de celui-ci.

10. Composé selon la revendication 1 qui est le 3-(1-{2-[4-(2-méthoxyphényl)pipérazin-1-yl]éthyl}-1,2,3,6-tétrahydropyridin-4-yl)-1H-indole ou un sel pharmaceutiquement acceptable de celui-ci.

11. Composé selon la revendication 1 qui est le 5-fluoro-3-(1-{2-[4-(2-méthoxyphényl)pipérazin-1-yl]éthyl}-1,2,3,6-tétrahydropyridin-4-yl)-1H-indole ou un sel pharmaceutiquement acceptable de celui-ci.

12. Composition pharmaceutique comprenant un support ou excipient pharmaceutiquement acceptable et un composé selon l'une quelconque des revendications 1 à 11, ou un sel pharmaceutiquement acceptable de celui-ci.

13. Composé selon l'une quelconque des revendications 1 à 11 ou un sel pharmaceutiquement acceptable de celui-ci pour l'utilisation comme spécialité pharmaceutique.

14. Utilisation d'un composé selon l'une quelconque des revendications 1 à 11 ou d'un sel pharmaceutiquement acceptable de celui-ci pour préparer un médicament pour l'utilisation thérapeutique comme inhibiteur du recaptage de la sérotonine.

15. Utilisation d'un composé selon l'une quelconque des revendications 1 à 11 ou d'un sel pharmaceutiquement acceptable de celui-ci pour préparer un médicament pour traiter la dépression.

16. Utilisation d'un composé selon l'une quelconque des revendications 1 à 11 ou d'un sel pharmaceutiquement acceptable de celui-ci pour préparer un médicament pour traiter l'anxiété.
